**Europäisches Patentamt**

**European Patent Office**

**Office européen des brevets**

(19)

(11) Veröffentlichungsnummer: **0 680 753 A2**

## (12) EUROPÄISCHE PATENTANMELDUNG

(21) Anmeldenummer: **95106926.9**

(22) Anmeldetag: **08.05.95**

(51) Int. Cl.⁶: **A61K 9/26, A61K 47/02**

(30) Priorität: **06.05.94 DE 4416003**
**06.05.94 DE 4416001**

(43) Veröffentlichungstag der Anmeldung:
**08.11.95 Patentblatt 95/45**

(84) Benannte Vertragsstaaten:
**DE ES FR GB IT**

(71) Anmelder: **Feinchemie GmbH Sebnitz**
**Höhenweg 9**
**D-01855 Sebnitz (DE)**

(72) Erfinder: **Böttcher, Horst,**
**Prof.Dr.rer.nat.habil.Dipl.Chem.**
**Ginsterstrasse 26**
**D-01196 Dresden (DE)**
Erfinder: **Kallies, Karl-Heinz, Oberingenieur**
**Götzinger Strasse 17**
**D-01855 Sebnitz (DE)**
Erfinder: **Marx, Jörg, Dr.rer.nat.Dipl.Chem.**
**Wasserstadt 36**
**D-06844 Dessau (DE)**

(54) **Metalloxidkomposit mit steuerbarer Wirkstoffabgabe.**

(57) Die Erfindung betrifft Komposite bestehend aus einer Metalloxidmatrix und mindestens einer darin aufgenommenen homogen, molekular-dispers und nicht-kristallin verteilten Komponente, Verfahren zu deren Herstellung sowie Verwendungsmöglichkeiten der Komposite.

Die inkorporierte Komponente kann durch einen Wirkstoff und gegebenenfalls einen oder mehrere Steuerstoffe gebildet werden.

Als Wirkstoffe finden flüssige oder feste Substanzen pflanzlichen oder organischen Ursprungs, die in lebenden Organismen eine biologische Wirkung hervorrufen, Verwendung.

Die Einbringung der Wirkstoffe in die Siliciumdioxid-Matrix erfolgt durch Sol-Gel-Technik.

Als Steuerstoffe dienen niedermolekulare wasserlösliche Stoffe, polyionische Verbindungen, mikroporöse Füllstoffe sowie hochsiedende Lösungsmittel.

Die Freisetzung von organischen Flüssigkeiten oder Ölen aus der Matrix kann entweder durch Penetrierungsmittel oder durch gezielte Temperaturerhöhung gesteuert werden.

Die erfindungsgemäßen Komposite finden Verwendung für die Herstellung von Arzneimittelzubereitungen, Kosmetika, Mitteln zur Körperpflege, bakteriostatischen und bakteriziden Mitteln, Insektiziden und Pestiziden.

EP 0 680 753 A2

Die Erfindung betrifft Komposite bestehend aus Metalloxid und mindestens einer darin aufgenommenen Komponente.

Die Erfindung betrifft insbesondere Komposite zur steuerbaren Wirkstoff-Freisetzung, die eine Siliciumdioxid-Matrix, in der das $SiO_2$ anteilig durch $Al_2O_3$ oder $MgO$ ersetzt sein kann, mindestens einen darin inkorporierten Wirkstoff und gegebenenfalls einen oder mehrere Steuerstoffe enthalten, ein Verfahren zu ihrer Herstellung sowie ihre Verwendung zur Herstellung von insbesondere Arzneimittelzubereitungen.

Die Erfindung betrifft ferner insbesondere Pulver und Filme aus Metalloxiden, die mindestens eine inkorporierte organische Flüssigkeiten oder Öle enthalten, Verfahren zu ihrer Herstellung sowie ihre Verwendung zur Herstellung von Arzneimittelzubereitungen, Kosmetika, Mittel zur Körperpflege, bakteriostatischen und bakteriziden Mitteln, Insektiziden und Pestiziden.

Die Erfindung kann insbesondere überall dort angewendet werden, wo die langsame Freisetzung einer Flüssigkeit oder eines Öls in das umgebende Medium oder der Schutz vor Verflüchtigung und Wechselwirkungen mit dem umgebendem Medium erforderlich sind.

Es ist bekannt, daß zur Verbesserung der mechanischen und chemischen Stabilität sowie zur Steuerung der Freisetzung pharmazeutische Wirkstoffe mit Hilfsstoffen gemischt oder überzogen werden. Im allgemeinen werden hierfür Gemische unterschiedlicher organischer Hilfsstoffe wie z.B. Kohlenhydrat-Abkömmlinge und/oder quellfähige hochpolymere Substanzen wie Gelatine oder Cellulose-Derivate verwendet. Ihre Nachteile bestehen besonders im Falle von Depotformen in einer stark milieu-abhängigen und darum schwierig zu beherrschenden Wirkstoff-Freisetzung im Magen-Darm-Trakt, wodurch die exakte Dosierung problematisch ist.

Weitere Probleme beim Einsatz organischer Hilfsstoffe ergeben sich u.a. aus deren unterschiedlicher Verträglichkeit beim Anwender sowie der Tatsache, daß optimale Eigenschaften nur durch Substanzgemische zu erreichen sind, die aufwendige Entwicklungsverfahren und Anwendungstests erfordern.

Zur Vermeidung der obengenannten Mängel gibt es seit einiger Zeit Bemühungen, anorganische Stoffe als Wirkstoff-Matrix zu nutzen. Unter den in Frage kommenden Stoffen nimmt Siliciumdioxid eine Sonderstellung ein; es ist billig, leicht zugänglich, nicht toxisch und stabil gegenüber Körperflüssigkeiten. Bei der Beladung des Siliciumdioxids mit Wirkstoff ergeben sich aber eine Reihe von Schwierigkeiten.

Geht man bereits von festen Siliciumdioxid-Teilchen aus und läßt den Wirkstoff an der Oberfläche der $SiO_2$-Partikel adsorbieren, z.B. durch Verwendung von feinem $SiO_2$-Pulver (JP 04159222A), kolloidalem $SiO_2$ (EP 487335-A1), mikroporösem Silicagel (EP 336014A) oder porösen Glaspartikeln (GB 2146531A) bzw. durch Verwendung von stabilisierten $SiO_2$-Dispersionen, stabilisiert durch polymere Säuren (EP 294206A), stabilisiert durch positiv geladene Detergenzien oder kationische Polymere (EP 478326A), ergeben sich aufgrund der herstellungsbedingt schwankenden Partikeloberfläche Probleme bei der reproduzierbaren Beladung der $SiO_2$-Partikel mit dem Wirkstoff. Außerdem sind die Möglichkeiten zur Herstellung hoher Wirkstoff-$SiO_2$-Verhältnisse begrenzt und die Möglichkeiten zur Einflußnahme auf die Wirkstoff-Freisetzung gering.

Eine günstigere alternative Variante besteht darin, lösliche $SiO_2$-Precursoren zum Einbau von Wirkstoffen zu nutzen. Als Precursoren werden Kieselsäure (DT 2030501, DT 2742912) bzw. Polyalkoxysiloxane und anschließende hydrolytische Polykondensation (DT 2642032) vorgeschlagen. Beide Varianten haben den Nachteil, daß sie nur auf eng begrenzte Wirkstoffklassen anwendbar sind und darum vorzugsweise für die Beschichtung von Wirkstoffen (Überzüge) genutzt werden. Im Falle des Einsatzes der alkalilöslichen Kieselsäure ist die Löslichkeit und die Stabilität des Wirkstoffs in verdünnten Alkali erforderlich. Die Anwendung von Polyalkoxysilanen erfordert die Verwendung hydrolysestabiler Wirkstoffe. Außerdem ist die Abwesenheit von Hydroxyl- und Aminogruppen erforderlich, um gegebenenfalls eine chemische Modifizierung des Wirkstoffstoffs zu verhindern. Diese Bedingungen erfüllen nur wenige pharmakologisch interessante Verbindungen.

Neue Möglichkeiten zur Einbringung von organischen Substanzen in eine Metalloxid-Matrix bietet die Sol-Gel-Technik. Dazu wird im EP 0439318 A2 ein Verfahren beschrieben, in dem Metallalkoxide in Gegenwart einer organischen Substanz hydrolysiert und polykondensiert werden, um dotierte Gläser für chemische Wechselwirkungen zu erhalten. Das Verfahren führt im Falle bioaktiver Wirkstoffe wie Enzymen zu starken Aktivitätsverlusten, da der Wirkstoff starr in der $SiO_2$-Matrix eingeschlossen ist; z.B. erfordern mit Glucoseoxidase beladenen Xerogel-Sensoren zur Glucosebestimmung inakzeptable Reaktionszeiten von 12 Min. (S.Braun et al., J.Non-Cryst. Solids 147/48 (1992) 739).

Erste Möglichkeiten zur Verbesserung der Reaktivität enzymbeladener Sol-Gel-Schichten durch den Einsatz von Penetrationsmitteln wurden in der deutschen Patentanmeldung P 4308146.0 (13.03.93) beschrieben. Während in der genannten Anmeldung Möglichkeiten zur Reaktivitätsverbesserung in der Schicht beschrieben wurden, müssen zur Nutzung von in Metalloxiden verkapselten pharmazeutischen Wirkstoffen in unterschiedlichen Arzneiformen Möglichkeiten gefunden werden, das Diffusionsverhalten des Wirkstoffs gezielt zu modifizieren. Derartige Möglichkeiten waren bislang nicht bekannt.

Es ist ferner bekannt, daß zahlreiche organische Flüssigkeiten oder leichtflüchtige Feststoffe für ihre Nutzung als pharmazeutische Wirkstoffe, Pestizide, Bakterizide, Sprengstoffe, Duftstoffe usw. in eine feste Form gebracht werden müssen, um eine der Anwendung angepaßte Handhabung zu sichern und eine ungesteuerte Verflüchtigung zu vermeiden.

Aus ökonomischen und ökologischen Gründen wird hierbei die Verwendung anorganischer Trägermaterialien, insbesondere von Metalloxiden, angestrebt. Hierfür wird meist die Adsorption von Flüssigkeiten an porösen Oberflächen genutzt. Allerdings weist die Methode, die z.B. im EP 143221 beschrieben wird, für zahlreiche Anwendungen einen großen Nachteil auf, indem die Flüssigkeiten in offenen Systemen sich allmählich wieder verflüchtigen, da sie nur physikalisch an der Feststoff-Oberfläche adsorbiert sind. Ein weiterer Nachteil besteht darin, daß die adsorbierten Substanzen ungeschützt dem Angriff von Atmosphärilien ausgesetzt sind und damit durch Oxidation oder Hydrolyse verändert werden können.

Es wurde darum intensiv nach Verkapselungsmöglichkeiten gesucht, die eine unerwünschte Verflüchtigung verhindern und eine Langzeitstabilität der Materialien sichern. Hierzu gibt es Vorschläge, die an einem Metalloxid-Pulver adsorbierten nichtwäßrigen Flüssigkeiten nachträglich mit einem filmbildenden Polymer wie Gelatine (NL 7214978, US 630928) zu verkapseln. Eine derartige mehrstufige Methode ist technologisch sehr kompliziert und erlaubt nur in beschränktem Maße eine kontrollierte Wiederfreisetzung der Flüssigkeit, da eine gleichmäßige Verkapselung der mit Flüssigkeit beladenen Metalloxidpartikel schwierig ist und Desorptionsprozesse durch Wechselwirkungen sowohl mit dem Polymer als auch mit dem Lösungsmittel während der nachträglichen Polymerumhüllung ablaufen können.

Eine universelle Methode zur Einkapselung von Flüssigkeiten in Metalloxide, die technologisch einfach und reproduzierbar ist, eine hohe Langzeitstabilität der inkorporierten Flüssigkeiten gewährleistet und ggf. die Steuerung der Freisetzung aus der Matrix gestattet, steht derzeit nicht zur Verfügung.

Es bestand also die Aufgabe, neue, verbesserte Möglichkeiten zur Inkorporation von Wirkstoffen in eine Metalloxid-Matrix zu finden, die mit einfachen und wenigen Hilfsmitteln realisierbar ist, und Verwendungen für derartige Komposite anzugeben.

Diese Aufgabe wird durch das Komposit mit den Merkmalen des Anspruchs 1 und durch Verfahren mit den Merkmalen gemäß einem der Ansprüche 16, 19 oder 20 sowie durch die in den Ansprüchen 21 und 22 angegebenen Verwendungen gelöst. Vorteilhafte Ausführungsformen ergeben sich aus den Unteransprüchen.

Es bestand insbesondere die Aufgabe, neue Möglichkeiten zur Inkorporation von Wirkstoffen in eine Siliciumdioxid-Matrix zu finden, die mit einfachen und wenigen Hilfsmitteln realisierbar sind, und eine gezielte Steuerung der Wirkstoff-Freisetzung gestatten.

Überraschenderweise konnte dieser Aspekt mit einfachen Mitteln erfindungsgemäß dadurch gelöst werden, daß die Wirkstoffe in neuartiger Weise in Gegenwart eines oder mehrerer Steuerstoffe in eine modifizierte Siliciumdioxid-Matrix inkorporiert werden, wodurch Wirkstoff-Siliciumoxid-Komposite mit steuerbarer Wirkstoff-Freisetzung erhalten werden.

Es bestand ferner die Aufgabe insbesondere darin, neue Möglichkeiten zur Inkorporation von Flüssigkeiten und Ölen in Metalloxidmatrizen zu finden, die mit einfachen und wenigen Hilfsmitteln realisierbar sind, eine hohe Stabilität der inkorporierten Flüssigkeiten und Öle gewährleisten, und ggf. die steuerbare Freisetzung der Flüssigkeitenund Öle aus der Metalloxidmatrix gestatten.

Überraschenderweise konnte dieser Aspekt mit einfachen Mitteln erfindungsgemäß dadurch gelöst werden, daß die organischen Flüssigkeiten und Öle durch einen Sol-Gel-Prozeß in die Metalloxide inkorporiert werden.

Es zeigte sich, daß unerwarteterweise organische Flüssigkeiten und Öle nach diesem Verfahren bis zu hohen Gewichtsanteilen in die Metalloxidmatrix eingebaut werden und dabei stabile, nichtklebrige Produkte (Pulver, Filmschichten) resultieren, die die organischen Flüssigkeiten und Öle in eingekapselter Form enthalten.

Im folgenden werden Ausführungsformen der Erfindung anhand von zwei Ausführungsbeispielen erläutert.

## Ausführungsbeispiel 1

Gegenstand der Erfindung sind gemäß einer Ausführungsform Komposite zur steuerbaren Wirkstoff-Freisetzung, die eine Siliciumoxid-Matrix, in der das $SiO_2$ anteilig durch $Al_2O_3$ oder $MgO$ ersetzt sein kann, mindestens einen darin inkorporierten Wirkstoff und gegebenenfalls einen oder mehrere Steuerstoffe enthalten.

Die Herstellung der Komposite ist dadurch gekennzeichnet, daß der Wirkstoff ggf. in Gegenwart eines Steuerstoffes in einem wäßrig-organischen $SiO_2$-Sol gelöst oder dispergiert wird und direkt oder nach Gelierung durch Lösungsmittelentfernung in ein Wirkstoff-Komposit überführt wird. Dadurch wird der Wirkstoff homogen und im Falle gelöster Wirkstoffe nichtkristallin und weitgehend molekular-dispers in die $SiO_2$-Matrix inkorporiert. Die Wahl der Herstellungsbedingungen (z.B. $SiO2$/Wirkstoff-Verhältnis) und der Zusatz von geeigneten Steuerstoffen beeinflussen entscheidend das Quell- und Diffusionsverhalten der $SiO_2$-Matrix und ermöglichen so die gezielte Steuerung der Wirkstoff-Freisetzung.

Die so hergestellten Komposit-Zubereitungen weisen eine Reihe von Vorteilen auf. Sie sind einfach herstellbar und ermöglichen eine steuerbare Wirkstoff-Freisetzung. Zur Herstellung werden keine komplizierten Apparate und Technologien benötigt. Die Technologie ist auf auf eine große Anzahl von Wirkstoffen anwendbar, da es praktisch keine Löslichkeitsrestriktionen gibt.

Die Komposit-Herstellung erfolgt in neutralen Lösungen bei moderaten Temperaturen (unter 5O°C), und darum können auch sehr hydrolyse- oder temperaturempfindliche Wirkstoffe verwendet werden. Es können Komposite mit beliebigen Wirkstoffanteilen erzeugt werden.

Die Komposit-Zubereitungen enthalten mit Ausnahme der nicht toxischen Steuerstoffe keine anderen Hilfsstoffe; dadurch wird eine ausgezeichnete Verträglichkeit erreicht. Die Wirkstoff-Liberation ist durch die Technologie und den Zusatz von Steuerstoffen in weiten Grenzen regelbar, und es können sehr unterschiedliche Freisetzungsverläufe erzielt werden. Die erhaltenen Zubereitungen können problemlos zu Arzneiformen weiterverarbeitet werden, sofern sie nicht schon allein eine Arzneiform darstellen.

Die erfindungsgemäßen Komposite können in Abhängigkeit vom gewünschten Freisetzungsverhalten in vielfältiger Weise modifiziert werden.

Als Ausgangsmaterial dient ein Siliciumdioxid-Sol in einem wäßrig-organischen Lösungsmittel, welches in bekannter Weise (vgl. C.J.Brinker, G.W.Scherer, Sol-Gel Science, Academic Press Inc., San Diego 1990) durch sauer oder basisch katalysierte Hydrolyse eines Tetraalkoxysilans erhalten wird; der organische Rest R kann dabei nahezu beliebig gewählt werden.

$$Si(OR)_4 \ + \ 2\,H_2O \ \xrightarrow{\text{(Kat)}} \ (SiO_2)_{n,Sol} \ + \ 4\,ROH \quad (1)$$

Bestimmte ökonomische Vorteile bietet die Hydrolyse kommerzieller teilhydrolysierter Tetraalkoxysilane (Polyalkoxysilane). Die durch Säuren oder Basen katalytisierte Hydrolyse wird vorrangig bei Raumtemperatur in einem mit Wasser mischbaren organischen Lösungsmittel durchgeführt und kann entsprechend der Löslichkeit des Wirkstoffs variiert werden, z.B. ist es im Falle wasserunlöslicher Wirkstoffe günstig, ein wasserarmes oder weitgehend wasserfreies $SiO2$-Sol und aprotische Lösungsmittel wie Aceton, Dioxan, Dimethylformamid oder höhersiedende Alkohole wie Methoxypropanol, Ethylglykol zu verwenden, damit bei der Lösungsmittelentfernung das Wasser gleichzeitig oder vor dem organischen Lösungsmittel entfernt wird. Diese neuartige Verfahrensweise (wasserarmes Sol, höher siedendes, organisches Lösungmittel) ist eine wesentliche Voraussetzung dafür, daß wasserunlösliche Wirkstoffe tatsächlich nichtkristallin in die $SiO2$-Matrix eingebaut werden.

Beispielsweise zeigen Carbamazepin/$SiO_2$-Komposite, die unter Verwendung eines $SiO_2$-Sols in Ethylglykol-Wasser hergestellt wurden, im Röntgendiffraktogramm der trockenen Substanz nur den für amorphe Substanzen typischen erhöhten Untergrund; dagegen wurden bei Verwendung von $SiO_2$-Solen in Ethanol-Wasser deutliche Interferenzen von Carbamazepin-Kristalliten beobachtet. Allerdings kann man auch $SiO_2$-Sole in Ethanol-Wasser zur Verkapselung wasserunlöslicher Wirkstoffe nutzen, wenn man diesen höhersiedende Substanzen (Kp. über 100°C) zumischt oder die Trocknung des wirkstoffhaltigen $SiO_2$-Sols so schnell durchführt (z.B. Sprühtrocknung), daß man Entmischungen während der Lösungsmittelentfernung verhindert. Die durch Sprühtrocknung erhaltenen Carbamazepin-$SiO_2$-Komposite bestanden aus ca. 20 μm großen kugelförmigen Partikeln, deren Röntgendiffraktogramm wiederum nur den für amorphe Substanzen typischen erhöhten Untergrund zeigte.

Der $SiO_2$-Gehalt im Sol liegt üblicherweise in einem Bereich zwischen 1-30 Gew.-% $SiO_2$ und wird vorrangig von ökonomischen Faktoren beeinflußt.

Auch die Verwendung gemischter Metalloxide, z.B. $SiO_2/Al_2O_3$ oder $SiO2/MgO$ ist problemlos möglich, wenn man vor der Hydrolyse die entsprechenden Metallalkoholate im gewünschten molaren Verhältnis dem Tetraalkoxysilan zumischt.

4

Die Hydrolyse kann durch verdünnte Mineralsäuren, z.B. 0,01 M Salzsäure, organische Säuren wie reine oder verdünnte Essigsäure, oder durch basische Substanzen wie wäßriger oder gasförmiger Ammoniak bzw. verdünnte Natronlauge oder Sodalösung katalysiert werden. In einigen speziellen Fällen kann die Hydrolyse mit den Hydrochloriden schwach basischer Wirkstoffbasen katalysiert werden.

Die Einbringung des Wirkstoffs in die $SiO_2$-Matrix geschieht durch Mischen des gelösten Wirkstoffs mit dem $SiO_2$-Sol oder durch Lösen des festen Wirkstoffs im Sol. Im Falle sehr schwer löslicher Wirkstoffe ist auch die Dispergierung in einem Homogenisator möglich. Das Gewichtsverhältnis Wirkstoff : $SiO_2$ liegt im allgemeinen bei 0,05... 1 : 1; bei speziell zu dosierenden Wirkstoffen kann dieses Verhältnis problemlos unter- oder überschritten werden.

Die erfindungsgemäßen Komposite können in Abhängigkeit vom erwünschten Freisetzungsverhalten in verschiedener Weise abgewandelt werden. Hierbei wurden drei wesentlichen Einflußparameter auf das Freisetzungsverhalten beobachtet:

(a) Wirkstoff-$SiO_2$-Verhältnis

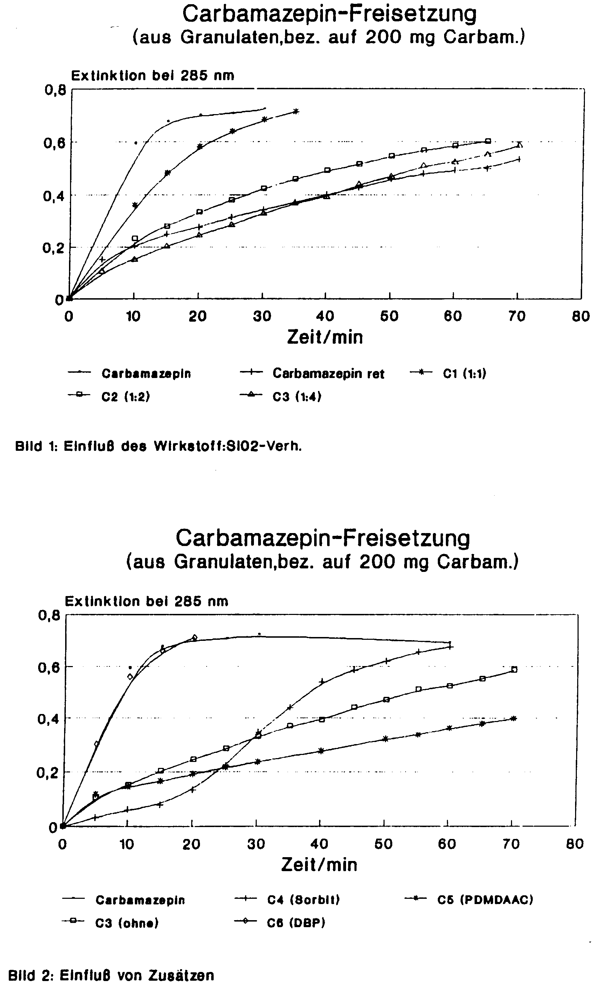

Erwartungsgemäß sinkt die Freisetzungsrate mit sinkendem Wirkstoff-$SiO_2$-Verhältnis (vgl. Bild 1)

(b) Kondensationsbedingungen

Es besteht die Möglichkeit, durch pH-Änderung oder Erwärmen das mit Wirkstoffen (W) beladene $SiO_2$-Sol zu einem Gel zu kondensieren:

$$(SiO_2)_{n,Sol} + W \text{--------}> (SiO_2)_{m,Gel}\text{-W} \quad m > n \qquad (2)$$

Im Falle der pH-Änderung führt die Einstellung des sauer oder basisch katalysierten $SiO_2$-Sols auf den Neutralpunkt (pH 7) in kurzer Zeit zur Erstarrung infolge Gelbildung. Das Gel enthält den Wirkstoff in homogener Verteilung. Durch Entfernung der Lösungsmittel aus dem Gel mit Hilfe üblicher Trockenverfahren erhält man pulverförmige Wirkstoff-$SiO_2$-Komposite (Gel-Variante). In einer anderen Variante werden die Lösungsmittel der schwach sauren oder alkalischen, mit Wirkstoff beladene Sollösung direkt durch Destillation oder Sprühtrocknung entfernt (Sol-Variante). Beide Varianten lassen sich mit Erfolg zum Einbau des Wirkstoffs in die $SiO_2$-Matix nutzen.

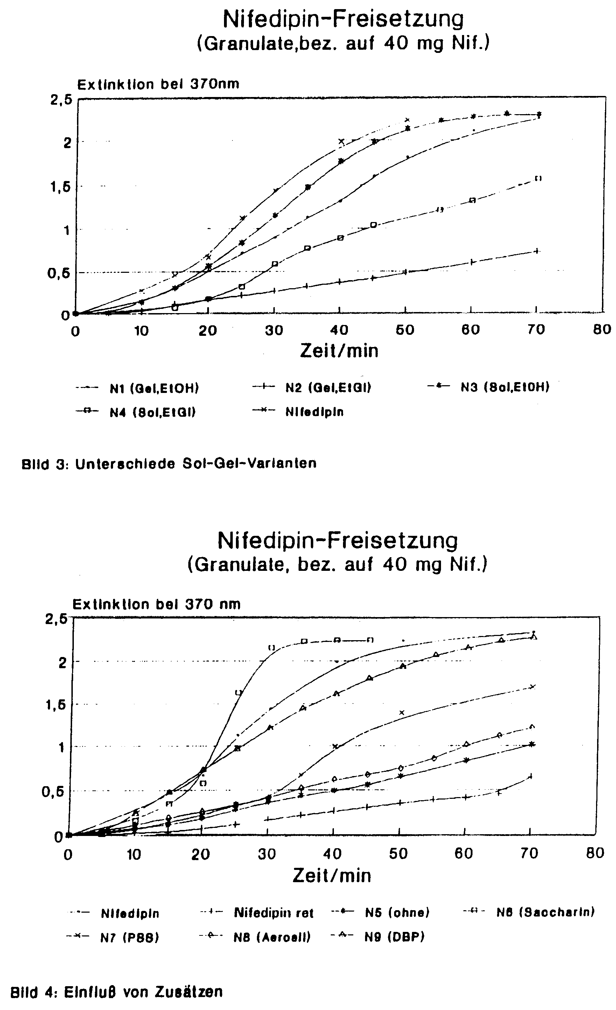

In den von uns untersuchten Beispielen führte die Variante Gelbildung/Trocknung stets zu einer wirksameren Wirkstoffverkapselung und einer langsameren Freisetzung als bei der direkten Trocknung des Sols (vgl. Bild 3). Allerdings wird in beiden Varianten das Freisetzungsverhalten zusätzlich durch die Art und die Bedingungen der Trocknung in reproduzierbarer Weise beeinflußt. Allgemein wurde beobachtet, daß eine Erhöhung der Trocknungstemperaturen oder eine thermische Nachbehandlung des Wirkstoff-Komposits die Wirkstoff-Freisetzung verlangsamt.

(c) Zusatz von Steuerstoffen

Eine besonders wirksame Steuerung des Freisetzungsverhaltens erreicht man durch den Zusatz von Steuerstoffen zur $SiO_2$-Sol-Wirkstoff-Lösung in Gewichtsanteilen von 1...50 % bezogen auf $SiO_2$. Überraschend wurde beobachtet, daß eine veränderte Wirkstoff-Freisetzung aus der starren $SiO_2$-Matrix durch Stoffe erreicht werden kann, die durch Herauslösen, Quellung oder Diffusionshemmung die Permeabiltät der Matrix modifizieren.

Es wurde gefunden, daß für diesen Zweck 4 Arten von Steuerstoffen geeignet sind:
(1) niedermolekulare, wasserlösliche Stoffe wie Saccharid-Derivate, z. B. Glucose, Lactose, Sorbit, Mannit oder Saccharose, Salze odfer Amide organischer Säuren, z.B. Salze der Benzoesäure oder Sorbinsäure, Amide wie o-Benzoesäuresulfimid (Saccharin), Acetamid oder Harnstoff sowie Ammoniumsalze wie Ammoniumsulfat oder Tetraalkyl-ammoniumchloride.

Derartige Steuerstoffe beschleunigen die Freisetzung aus der Matrix, da sie durch teilweises Herauslösen aus der Matrix deren Permeabilität erhöhen.
(2) polyanionische Verbindungen wie die Salze von Polystyren-sulfosäure, Polyacrylsäure, Carboxymethylcellulose, Dextransulfat oder Cellulosesulfat bzw. polykationiche Verbindungen wie Polydimethyldiallyl-ammoniumchlorid (PDMDAAC).

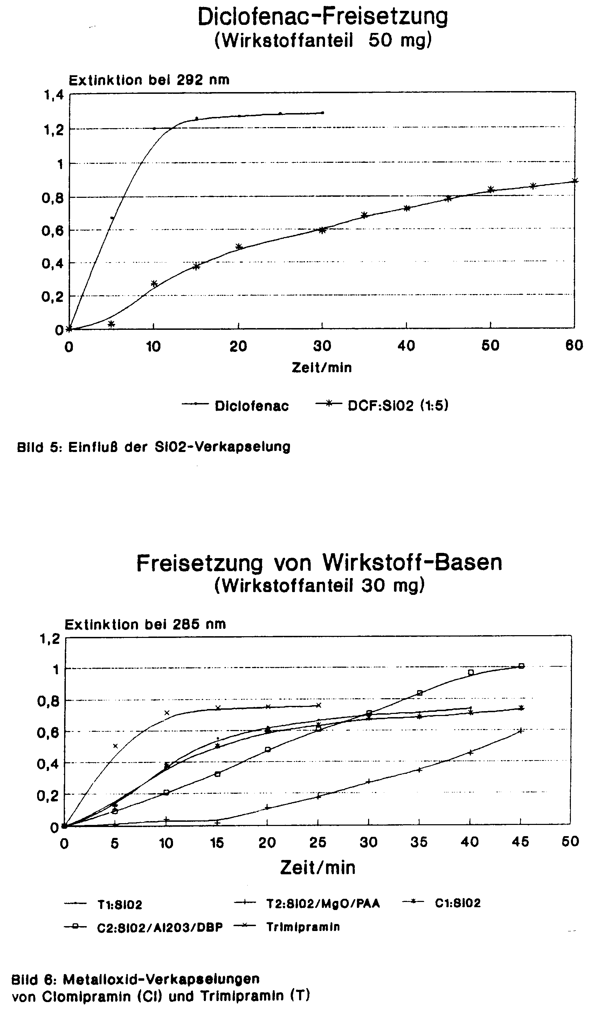

Die Steuerstoffe können die Freisetzung des Wirkstoffs aus der Matrix beschleunigen, indem sie deren Quelleigenschaften erhöhen. In speziellen Fällen ist aber auch eine Herabsetzung der Freisetzungsrate möglich, wenn die Wirkstoffe z.B. durch salzartige Wechselwirkungen in der Matrix fixiert werden (z.B. Polyacrylsäure/Trimipramin-Base, Bild 6)

(3) mikroporöse Füllstoffe wie feste oder dispergierte Aerosile.

Diese Steuerstoffe verbessern die Freisetzung, indem die Permeabilität der Matrix erhöht wird ("Dochtwirkung").

(4) hochsiedende Lösungsmittel wie Phthalsäurester, Glycerinether oder Paraffinöl,in denen der Wirkstoff gut löslich ist.

Es wurde überraschend gefunden, daß auch hochsiedende Flüssigkeiten und Öle als Steuerstoffe in die Matrix eingebaut werden können. Die erhaltenen Wirkstoff-Granulate sind nicht klebrig, sondern trocken und rieselfähig. Durch Zusatz von hochsiedenden Flüssigkeiten kann man die Wirkstoff-Freisetzung extrem verzögern oder beschleunigen in Abhängigkeit von den Löslichkeitsverhältnissen des Wirkstoffs in der hochsiedenden Flüssigkeit und dem umgebenden Medium.

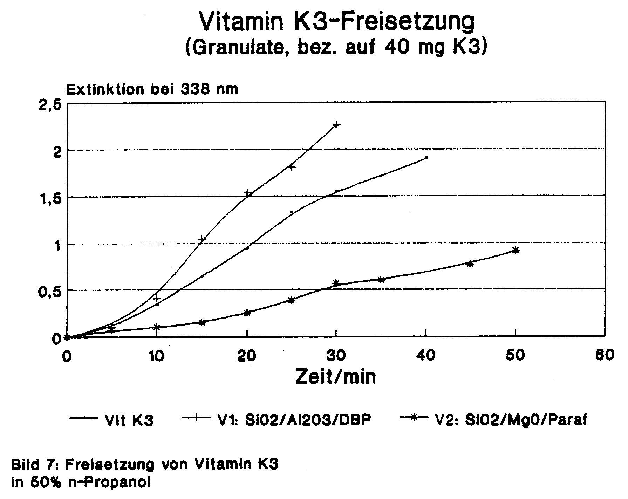

Beispielsweise kann das öllösliche Vitamin $K_3$ (Beispiel 1.6, Bild 7) unter Zusatz von Dibutylphthlat (DBP) oder Paraffinöl AB (Paraf) in die Metalloxid-Matrix eingebracht werden. In dem Freisetzungsmedium 50% n-Propanol ist DBP teilweise löslich, das führt zu einer starken Beschleunigung der Freisetzung, da DBP für den Wirkstoff gewissermaßen als Transportmittel aus der Matrix wirkt. Paraffinöle sind dagegen in wäßrigen Alkoholen kaum löslich, darum ist die Wirkstoff-Freisetzung stark verzögert, denn das Bestreben des öllöslichen Wirkstoffs in die wäßrig-alkoholische Phase zu diffundieren ist gering.

Wie aus den Beispielen ersichtlich ist (vgl.Bild 2 und 4), kann in Abhängigkeit von Art und Konzentration des zugesetzten Steuerstoffs die Wirkstoff-Freisetzung stark beschleunigt oder verlangsamt werden.

Durch Variation von Art und Konzentration der Steuerstoffe ist es durch wenige orientierende Versuche möglich, für einen speziellen Wirkstoff die Kompositzusammensetzung zu ermitteln, die unter den verwendeten Konzentrationsverhältnissen und Kondensationsbedingungen das erwünschte Freigabeverhalten zeigt. Vorteilhaft ist hierbei die über einen weiten Zeitbereich kontinuierliche Wirkstoff-Freisetzung.

Die erfindungsgemäß hergestellten Wirkstoff-Komposite können in verschiedener Weise genutzt werden. Besonders vorteilhaft ist die Anwendung als pharmazeutische Zubereitung mit steuerbarer Wirkstoff-Freisetzung. Hierzu können die Komposite direkt als Pulver, Granulat oder Tablette sowie in verkapselter, dragierter oder überzogener Form appliziert werden. Wie die Beispiele zeigen, gibt es hinsichtlich des Wirkstoffs keine erkennbaren Restriktionen, z.B. können neutrale, saure, basische, wasserlösliche oder wasserunlösliche Wirkstoffe verwendet werden, wie z.B. Alprazolam, Carbamazepin, Clonidin, Detajmiumbitartrat, Diclofenac, Diazepam, Glibenclamid, Medazepam, Metoclopramid, Nifedipin, Pentoxifyllin, Prazosin, Talinolol, Verapamil.

Das Verfahren gestattet auch, flüssige hochsiedende Wirkstoffe zu verkapseln. Das bietet zum Beispiel den Vorteil, anstelle von Hydrochloriden flüssige oder niedrigschmelzende Wirkstoff-Basen (z.B. die flüssige Clomipramin-Base oder die niedrig schmelzende Trimipramin-Base) in der $SiO_2$-Matrix zu verkapseln oder durch den Zusatz hochsiedender Hilfsstoffe wie Phthalsäureester, Glycerinether oder Paraffinöl das Freisetzungsverhalten des Wirkstoffs zu modifizieren. In gleicher Weise können auch fettlösliche oder ölige Vitamine wie Vitamin $K_3$, Vitamin A oder Vitamin E oder deren Gemische in die $SiO_2$-Matrix eingebracht werden.

Analog zu den pharmazeutischen Wirkstoffen können auch Wirkstoffe in die $SiO_2$-Matrix inkorporiert werden, wie sie in kosmetischen Zubereitungen, zur Schädlingsbekämpfung oder zum Pflanzenschutz verwendet werden, sofern eine modifizierte Wirkstoff-Freisetzung wünschenswert ist.

Aus der Fülle der Herstellungsvarianten sind einige in den folgenden Beispielen näher beschrieben.

**Beispiel 1.1:**

(a) Allgemeine Herstellung eines reinen $SiO_2$-Sols (Sol A)

50 ml Tetraethoxysilan, 200 ml Ethanol (Ethylglykol oder Dioxan) und 100 ml 0,01 **N** HCl wurden 20 Std. bei Raumtemperatur gerührt.

(Zur Anpassung an die Löslichkeit des Wirkstoffs können Art und Konzentrationsverhältnis des organischen Lösungsmittels in weiten Grenzen geändert werden. Außerdem können kommerzielle Teilhydrolysate (z.B. Polykieselsäureester Dynasil 40, Dynasil 220/Hüls AG) anstelle des Tetraethoxysilans eingesetzt werden.)

(b) Herstellung eines $SiO_2$-$Al_2O_3$-Sols (Sol B)

10 g Tetraethoxysilan und 2 g Aluminium-triisopropylat werden in 50 ml Ethylenglykolmonomethylether gelöst. Unter Rühren werden 5 ml 5 % wäßrige Ammoniak-Lösung zugetropft. Nach zwölf Stunden wird die klare Lösung zur Wirkstoff-Verkapselung eingesetzt.

(c) Herstellung eines $SiO_2$-MgO-Sols (Sol C)

12,7 g Tetraethoxysilan und 0,7 g Magnesiumethylat werden in 120 ml Ethanol gelöst. Unter Rühren werden 5 ml 5 % wäßrige Ammoniak-Lösung zugetropft. Nach zwölf Stunden wird von etwas weißem Niederschlag abfiltriert und das Filtrat zur Wirkstoff-Verkapslung eingesetzt.

**Beispiel 1.2:**

Carbamazepin-$SiO_2$-Komposite

Jeweils 1 g des Wirkstoffs Carbamazepin wurden durch leichtes Erwärmen in der entsprechenden Menge Sol gelöst und durch Zugabe weniger Tropfen 10%iger Natriumcarbonat-Lösung oder 5% iger Ammoniaklösung bis zum Erreichen des pH-Wertes 7 zum Gelieren gebracht. Das Gel wurde 3 Std. an der Luft und 12 Std. bei 80°C im Trockenschrank getrocknet (Gel-Variante).

Eine andere Variante besteht darin, die Wirkstoff-Sol-Lösung im Rotationsverdampfer auf dem Wasserbad (80°C) zur Trocknung zu bringen (Sol-Variante).

Die erhaltenen Granulate wurden röntgendiffraktometrisch untersucht und die Freisetzungskinetik des Carbamazepins in 1 % wäßriger Dodecylsulfatlösung in einer geeigneten Freisetzungsapparatur spektralphotometrisch bestimmt und mit der Kinetik des kommerziellen Retardpräparates sowie der des reinen Wirkstoffs verglichen, vgl. Bild 1,2.

Tabelle 1.1

| Rezepturen zur Herstellung unterschiedlicher Carbamazepin-$SiO_2$-Komposite (bezogen auf 1 g des Wirkstoffs Carbamazepin) | | | | | |
|---|---|---|---|---|---|
| Nr. | ml Sol A | Lösungsmittel | Wirkstoff:$SiO_2$ | Zusatz | Variante |
| C1 | 25 | Ethanol | 1:1 | - | Gel |
| C2 | 50 | Dioxan | 1:2 | - | Gel |
| C3 | 100 | Ethylglykol | 1:4 | - | Gel |
| C4 | 100 | " | 1:4 | 1.0 g Sorbit | Gel |
| C5 | 100 | " | 1:4 | 3 ml PDMDAAC(40%) | Gel |
| C6 | 100 | " | 1:4 | 2 g Dibutylphthalat | Gel |

**Beispiel 1.3:**

Nifedipin-$SiO_2$-Komposite

Einbringen des Wirkstoffs Nifedipin in die $SiO_2$-Matrix

Die Verfahrensweise ist analog Beispiel 1.1. Die Freisetzungskinetik der Granulate erfolgt in einer geeigneten Freisetzungsapparatur in 50 %iger Essigsäure. Die Ergebnisse werden mit der Freisetzungskinetik des reinen Wirkstoffs sowie der kommerziellen Retardform verglichen, vgl. Bild 3,4.

Die Resultate korrelieren mit den Freisetzungsergebnissen, die in Wasser/Ethanol (7 : 1) in einer Paddle-Apparatur (DAB 10) sowie in 0,1 N-Salzsäure in einer Durchflußzelle Typ A (DAB 10) erzielt werden.

Tabelle 1.2

| Rezepturen zur Herstellung unterschiedlicher Nifedipin-$SiO_2$-Komposite (bezogen auf 1 g des Wirkstoffs Nifedipin) | | | | | |
|---|---|---|---|---|---|
| Nr. | ml Sol A | Lösungsmittel | Wirkstoff:$SiO_2$ | Zusatz | Variante |
| N1 | 125 | Ethanol | 1:5 | - | Gel |
| N2 | 125 | Ethylglykol | 1:5 | - | Gel |
| N3 | 125 | Ethanol | 1:5 | - | Sol |
| N4 | 125 | Ethylglykol | 1:5 | - | Sol |
| N5 | 250 | Ethanol | 1:10 | - | Gel |
| N6 | 250 | " | 1:10 | 2g Saccharin-Na | Gel |
| N7 | 250 | " | 1:10 | 2g Na-Polystyren-sulfonat/Aldrich | Gel |
| N8 | 250 | " | 1:10 | 6 ml Aerosil-Dispersion K330 (Degussa) | Gel |
| N9 | 100 | Ethylglykol | 1:4 | 2g Dibutyl-phthalat | Gel |

**Beispiel 1.4:**

Diclofenac-$SiO_2$-Komposit

1g Diclofenac und 0,2 g Ammoniumsulfat werden in 125 ml $SiO_2$-Sol (in Wasser/Ethylglykol) unter Erwärmen gelöst; nach wenigen Minuten tritt Gelierung ein. Das Gel wird 3 Std. an der Luft und 12 Stunden bei 80° getrocknet. Die Freisetzungskinetik (vgl. Bild 5) wurde in 1 % wäßriger Dodecylsulfat-Lösung bestimmt.

**Beispiel 1.5:**

Wirkstoffbasen-$SiO_2$-Komposite

(a) 1g der flüssigen Wirkstoff-Base Clomipramin bzw. 1 g des niedrig schmelzenden Trimipramin werden in 62.5 ml $SiO_2$-Sol (in Wasser/Ethylglykol) und 0,2 g Glycerinmonohexylether gelöst. Beim Erwärmen tritt nach kurzer Zeit Gel-Bildung ein. Die Gele wurden 3 Std. an der Luft und 12 Std. bei 50°C im Trockenschrank getrocknet. Die Freisetzungskinetik der Wirkstoffe (vgl. Bild 6) wurde in 0,1n Salzsäure bestimmt und mit der von reinem Trimipramin verglichen (Kurven C1, T1 in Bild 6).
(b) 0,24 g Clomipramin und 0,24 g Dibutylphthalat werden in 20 ml Sol B unter leichtem Erwärmen gelöst. Die klare Lösung geliert beim Erstarren über Nacht. Das Gel wird drei Stunden an der Luft und zwölf Stunden im Trockenschrank bei ca. 60°C getrocknet (Freisetzungskurve C2 in Bild 6).
(c) 0,3 g Trimipramin werden in 50 ml Sol C gelöst und 3 ml einer 10 % wäßrigen Lösung von Polyacrylsäure 20 000 (Na-Salz; Fluka) zugesetzt. Die Weiterverarveitung erfolgt analog (b); die Freisetzungskurve T2 des Komposits ist in Bild 6 zu sehen.

**Beispiel 1.6:**

Vitamin $K_3$-Komposite

(a) 0,24 g Vitamin $K_3$ (Menadion, Merck) und 0,24 g Dibutylphthalat werden in 20 ml Sol B unter leichtem Erwärmen gelöst. Die klare Lösung geliert über Nacht. Das Gel wird drei Stunden an der Luft und drei Stunden im Trockenschrank unter Lichtausschluß getrocknet.
Die Bestimmung der Wirkstoff-Freisetzung erfolgte in n-Propanol/Wasser (50/50), vgl. Kurve V1 in Bild 7.
(b) 0,3 g Vitamin $K_3$ und 0,3 g Paraffinöl werden unter Erwärmen in 5 ml Methylglykol gelöst und dieses mit 50 ml Sol C gemischt. Die Weiterverarbeitung erfolgt analog (a); die Freisetzungskurve V2 des Wirkstoff-Komposits ist in Bild 7 zu sehen.

**Ausführungsbeispiel 2**

Zur Herstellung von Pulvern oder Filmschichten wird eine zu inkorporierende Flüssigkeit in einem organisch-wäßrigen Metalloxid-Sol gelöst, das man durch saure oder alkalische Hydrolyse von Metallalkoxiden (vorzugsweise von Silicium-, Aluminium- oder Titanalkoxiden oder deren Gemische) in einem mit Wasser mischbaren organischen Lösungsmittel in bekannter Weise erhält, vgl. C.J.Brinker, G.W.Scherer: Sol-Gel-Science, Academic Press, Inc., San Diego, 1990.

In speziellen Fällen können auch Sole verwendet werden, die aus Metalloxiden und alkylierten Metalloxiden bestehen (und z.B. durch gemeinsame Hydrolyse von Tetraalkoxysilanen und Trialkoxyalkylsilanen gebildet werden.) Die einzukapselnden Flüssigkeiten können dabei vor, während oder nach der Herstellung des Metalloxid-Sols zugesetzt werden. Die Anforderungen an die Flüssigkeit bestehen lediglich darin, daß sie sich in dem Sol lösen und ihr Siedepunkt hinreichend über dem Siedepunkt des im Sol enthaltenen Lösungsmittels, vorzugsweise über 100 °C bei Normaldruck ($10^5$ Pa) liegt. Das Gewichtsverhältnis Flüssigkeit : Metalloxid liegt vorzugsweise bei 0,01...0,3 : 1, wobei Über- oder Unterschreitungen möglich sind.

Restriktionen bezüglich der chemischen Struktur der Flüssigkeiten sind nicht erkennbar. Es sind problemlos Flüssigkeitsgemische als auch Flüssigkeiten natürlicher Herkunft (ätherische Öle, Pflanzenextrakte, Tiersekrete, Pheromone) verwendbar. Da der Herstellungsprozeß weitgehend im neutralen pH-Bereich bei moderaten Temperaturen (unter 50 °C) abläuft, sind auch hydrolyse- oder temperaturempfindliche Flüssigkeiten einsetzbar. Der Wassergehalt des Metalloxid-Sols sowie die Art des mit Wasser mischbaren organischen Lösungsmittel kann entsprechend der Löslichkeit der einzukapselnden Flüssigkeit variiert werden, z.B. ist es für wasserunlösliche Flüssigkeiten und Öle günstig, ein wasserarmes oder weitgehend wasserfreies Metalloxid-Sol und aprotische Lösungsmittel wie Aceton, Dioxan, Dimethylformamid, oder höhersiedende Alkohole wie Methoxypropanol, Ethylglykol zu verwenden, damit bei der Lösungsmittelentfernung das Wasser gleichzeitig oder vor dem organischen Lösungsmittel entfernt wird.

Die mit höhersiedenden Flüssigkeiten und Ölen beladenen Metalloxid-Sole können nun in verschiedener Weise in einen Feststoff umgewandelt werden:

(a) das Sol kann durch schonende Trocknung direkt in ein Metalloxid-Pulver mit eingekapselter Flüssigkeit umgewandelt werden. Diese Methode ist dann vorteilhaft, wenn sehr wasserarme Metalloxid-Sole eingesetzt werden und eine erhebliche Differenz der Siedepunkte (über 50 °C) zwischen Sollösungsmittel und einzukapselnder Flüssigkeit besteht.

(b) das mit einzukapselnder Flüssigkeit beladene Sol wird durch Erwärmen oder pH-Änderung (Einstellung des sauren oder basischen Metalloxid-Sols auf den Neutralpunkt) in ein Gel umgewandelt. Das erstarrte Gel enthält die einzukapselnde Flüssigkeit in homogener Verteilung. Durch Entfernen des Lösungsmittels mit Hilfe üblicher Trocknungsverfahren erhält man trockene, rieselfähige Pulver, in denen die Flüssigkeit inkorporiert ist.

(c) das mit einzukapselnder Flüssigkeit beladene Metalloxid-Sol kann durch übliche Beschichtungsverfahren (z.B. Eintauchen, Begießen, Aufschleudern) ggf. unter Zusatz eines haftverbessernden Netzmittels auf einen beliebigen Schichtträger (z.B. Polymer- oder Metallfolie, Papier, textiles Gewebe oder Holz) aufgebracht und getrocknet werden. Man erhält transparente Metalloxidfilme, in denen die höhersiedende Flüssigkeit inkorporiert ist.

Der Nachweis der inkorporierten Flüssigkeit in den Metalloxidpulvern und -filmen kann durch thermische oder extraktive Freisetzung aus der Metalloxidmatrix und nachfolgende spektroskopische Identifikation erfolgen, vgl. Beispiele.

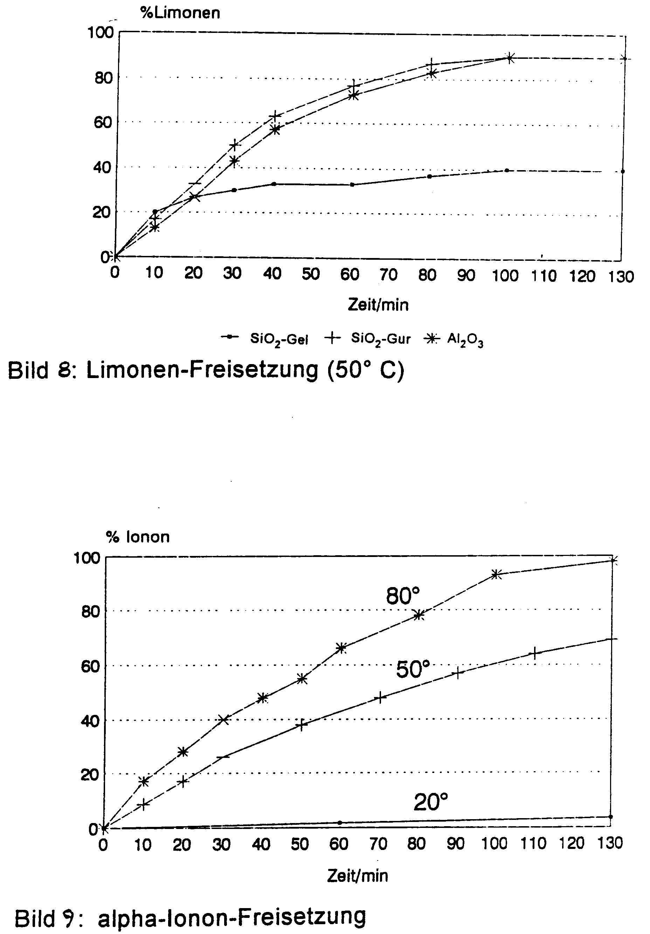

Wie derartige Untersuchungen zeigen, sind die Flüssigkeiten in der Metalloxidmatrix stabil eingeschlossen. Die Verflüchtigungsrate nach dem erfindungsgemäßen Verfahren ist viel geringer als bei den an porösen Metalloxidschichten adsorbierten Flüssigkeiten, vgl. Bild 8.

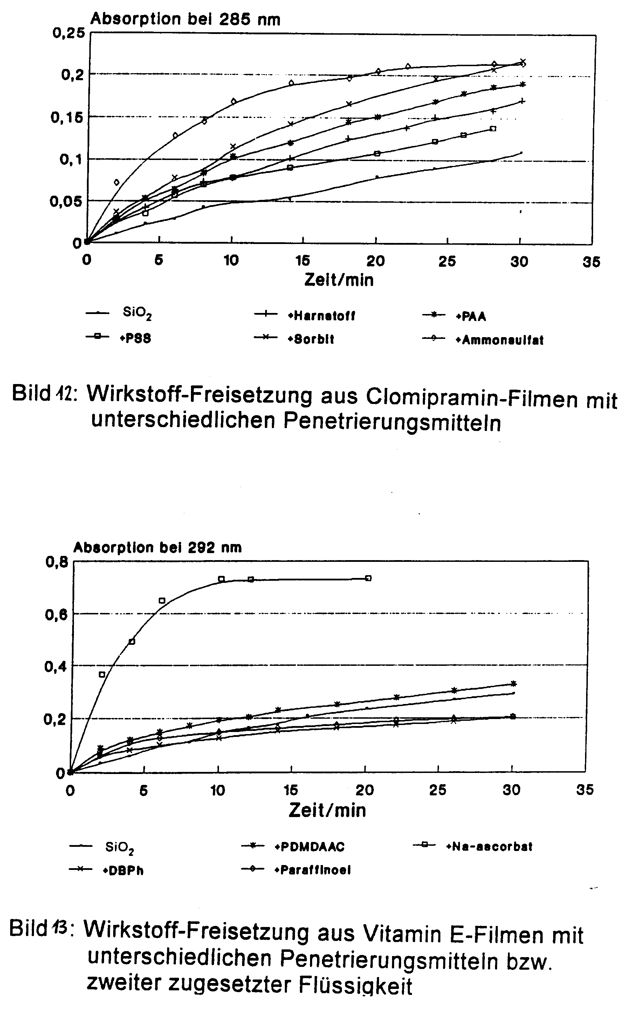

Für zahlreiche Anwendungen ist es erforderlich, die Flüssigkeit aus der Metalloxidmatrix gezielt freizusetzen. Das ist auf thermischen oder extraktiven Wege möglich. Während bei Raumtemperatur die Verflüchtigung der eingekapselten Flüssigkeit sehr gering ist, steigt bei Temperaturerhöhung in Abhängigkeit vom Dampfdruck der Flüssigkeit die Freisetzungsrate, vgl. Bild 9. Dadurch kann man die Freisetzung der eingekapselten Flüssigkeit in die umgebende Gasphase steuern. Die Freisetzung der Flüssigkeit aus der Metalloxidmatrix in eine umgebende flüssige Phase kann neben der Wahl des Metalloxid-Sols (vgl. Bild 11) vorrangig durch den Einsatz von Penetrierungsmitteln erreicht werden, die vor, während oder nach der Herstellung des Metalloxid-Sols in Gewichtsanteilen bis zu 100 % bezogen auf das Metalloxid zugesetzt werden. Die Penetrierungsmittel beschleunigen wie in Bild 12 gezeigt die Freisetzung der inkorporierten Flüssigkeit aus der Metalloxidmatrix, indem sie durch Herauslösen oder Quellung die Durchlässigkeit der Matrix erhöhen. Als besonders geeignete Penetrierungsmittel erwiesen sich niedermolekulare wasserlösliche Stoffe wie Saccharid-Derivate, Salze oder Amide organischer Säuren oder Ammoniumsalze sowie wasserlösliche polyanionische Verbindungen wie die Salze der Polystyrensulfosäure, Polyacrylsäure oder Carboxymethylcellulose oder polykationische Verbindungen wie Polydimethyldiallylammoniumchlorid.

Zur Steuerung der Freisetzung kann auch eine zweite Flüssigkeit zugesetzt werden. In Abhängigkeit von der Löslichkeit des flüssigen Wirkstoffs in der zugesetzten Flüssigkeit und der umgebenden Lösungsphase kann eine verzögerte oder beschleunigte Freisetzung eintreten. Bild 13 zeigt z.B. den Fall, wo der Zusatz hoch-siedender Flüssigkeiten wie Dibutylphthalat oder Paraffinöl die Freisetzung des öllöslichen flüssigen Vitamin E verzögern.

Das erfindungsgemäße Verfahren zeichnet sich dadurch aus, daß mit einer sehr einfachen Technologie praktische beliebige organische Flüssigkeiten, deren Siedepunkt hinreichend über dem Siedpunkt des im Sol enthaltenden Lösungsmittels liegt, und Öle effektiv verkapselt werden können. Die erfindungsgemäß hergestellten Pulver und Filme sind feste Produkte, in denen die organischen Flüssigkeiten und Öle bei Raumtemperatur langzeitstabil inkorporiert sind. Die Freisetzung der inkorporierten Flüssigkeiten und Öle kann thermisch oder durch den Zusatz von Penetrierungsmittel gesteuert werden.

Anwendungen der erfindungsgemäß hergestellten Metalloxidpulver und Filme mit inkorporierten organischen Flüssigkeiten und Ölen ergeben sich z.B. zur Herstellung

- fester oder dünnschichtiger Darreichungsformen in der Pharmazie bei Verwendung flüssiger oder öliger Wirkstoffe und Vitamine auf natürlicher oder synthetischer Grundlage. Spezielle Anwendungsformen dünnschichtiger Darreichungsformen sind hierbei transdermale Systeme mit flüssigen oder öligen Wirkstoffen (z.B. Nitroglycerin). Durch den Zusatz spezieller Penetrationsmittel (Harnstoff, Öle wie Glycerinether) kann der Transport des Wirkstoffs durch die Haut gefördert werden.
- fester oder dünnschichtiger Darreichungsformen in der Kosmetik und Körperpflege bei Verwendung flüssiger oder öliger Riechstoffe und Hautpflegemittel auf natürlicher und synthetischer Grundlage
- fester oder dünnschichtiger Darreichungsformen für desinfizierende Zwecke (z.B. Schädlingsbekämpfung, Pflanzenschutz) bei Verwendung flüssiger oder öliger Pheromone, Bakterizide oder Pestizide auf natürlicher oder synthetischer Grundlage.
- von verkapselten Duftstoffen und Lockstoffen.

Aus der Fülle der Herstellungsvarianten sind einige in den folgenden Beispielen näher beschrieben.

**Beispiel 2.1:**

**Herstellung der Metalloxid-Sole**

(A) $SiO_2$-Sol

50 ml Tetraethoxysilan, 200 ml Ethanol (Ethylglykol, Dioxan) und 100 ml 0,01 N Salzsäure (oder 5 % Ammoniak) werden 20 Std. bei Raumtemperatur gerührt.

(B) Sol aus $SiO_2/MeSiO_x$

35 ml Tetraethoxysilan, 15 ml Trimethoxymethylsilan werden in 200 ml Ethanol und 100 ml 0,01 N Salzsäure 20 Std. bei Raumtemperatur gerührt.

(C) Sol aus $SiO_2/TiO_2$

Zu 45 ml Tetraethoxysilan, 5 ml Tetraisopropylorthotitanat in 200 ml Ethanol werden bei Raumtemperatur langsam 10 ml 0,1 N Salzsäure zugetropft und 20 Std. gerührt.

(D) Sol aus $SiO_2/Al_2O_3$

In 45 ml Tetraethoxysilan und 200 ml Ethanol werden unter Erwärmen 5 g Aluminiumtriisopropylat gelöst. Anschließend werden bei Raumtemperatur unter Rühren 10 ml 0,1 Salzsäure langsam zugetropft. Die klare Lösung beginnt nach ca. 3 Std. langsam zu gelieren. Sie wird dann unmittelbar weiterbearbeitet.

**Beispiel 2.2:**

**Verkapselung der Riechstoffe Limonen, Ionon,Famesol sowie der Hochsieder Dibutylphthalat und Paraffinöl**

Jeweils 1 g der Flüssigkeit werden in 50 ml sauer hydrolysiertem $SiO_2$-Sol A (in Wasser/Ethylglykol) gelöst und durch leichtes Erwärmen und Zutropfen von 5%igem Ammoniak geliert. Das erstarrte Gel wird anschließend 20 Std. an der Luft getrocknet und ergibt helle, glasartige Pulver.

Ein Vergleich der Freisetzung von 1g Limonen, adsorbiert an 5 g Kieselgur/Fluka bzw. 5 g $Al_2O_3$-Pulver/Laborchemie Apolda, ist mit erfindungsgemäß inkorporierten Limonen ($SiO_2$-Gel) in Bild 8 zu sehen.

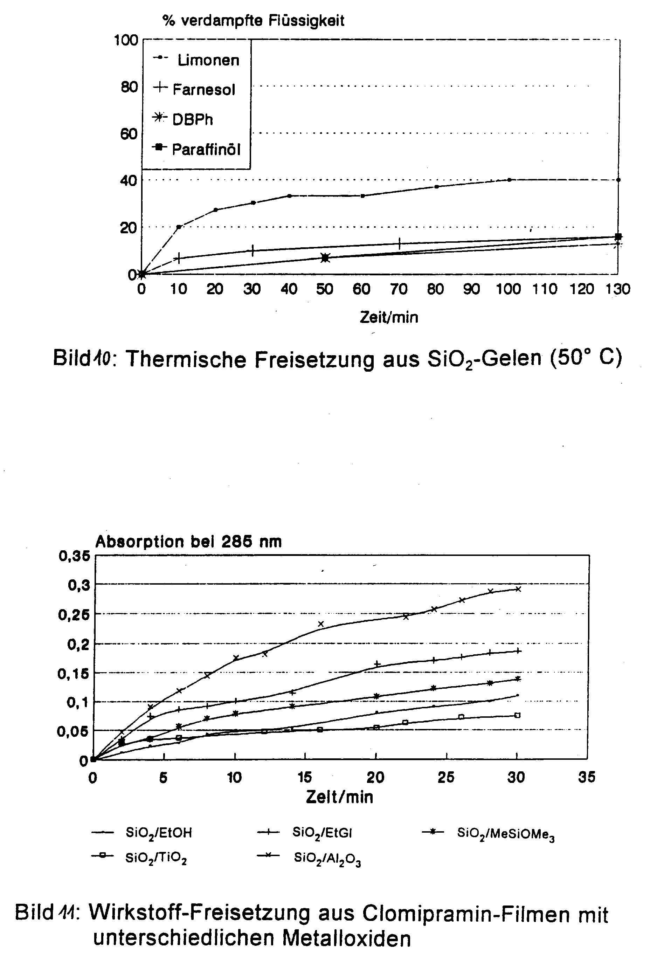

Die Freisetzung von erfindungsgemäß inkorporiertem alpha-Ionon aus der $SiO_2$-Matrix bei 20, 50 und 80°C innerhalb von 130 min. ist in Bild 9 aufgeführt. Die Freisetzung der oben aufgeführten organischen Flüssigkeiten und Öle beim Erwärmen auf 50°C ist in Bild 10 zu sehen.

**Beispiel 2.3:**

**Metalloxid-Filme mit inkorporierter flüssiger Wirkstoff-Base Clomipramin**

Es wird eine Stammlösung aus 1g Clomipramin und 10 ml Ethylglykol hergestellt ("Stammlösung"). 10 ml Metalloxid-Sol und 1 ml Stammlösung werden ggf. unter Zusatz eines Steuerstoffs gelöst und die klare Lösung auf Filmunterlage (140 $\mu$m Acetylcellulose) aufgetragen. Nach Trocknen an der Luft erhält man klare Filmschichten.

Die Freisetzung des Clomipramins aus der Metalloxidmatrix wurde in folgender Weise bestimmt:

1x3 $cm^2$ beschichtete Folie wurden in einer Küvette mit 3 ml Phosphatpuffer pH 6.8 behandelt und die Konzentrationen des freigesetzten Wirkstoffs Clomipramin in Abhängigkeit von der Zeit spektralphotometrisch (im Maximum bei 285 nm) bestimmt.

## Tabelle 2.1: Rezepturen

### (a) Unterschiedliche Metalloxid-Sole (vgl. Bild 11)

| | |
|---|---|
| $SiO_2$/EtOH: | 1 ml Stammlösung + 10 ml Sol A in Ethanol |
| $SiO_2$/EtGl: | 1 ml Stammlösung + 10 ml Sol A in Ethylglykol |
| $SiO_2$/MeSiOMe$_3$: | 1 ml Stammlösung + 10 ml Sol B |
| $SiO_2$/TiO$_2$: | 1 ml Stammlösung + 10 ml Sol C |
| $SiO_2$/Al$_2$O$_3$: | 1 ml Stammlösung + 10 ml Sol D |

### (b) Zusatz unterschiedlicher Penetrierungsmittel (vgl. Bild 12)

| | | |
|---|---|---|
| $SiO_2$: | 1 ml Stammlösung + 10 ml Sol A in Ethanol | |
| Harnstoff: | wie oben, | + 0,1 g Harnstoff |
| PAA : | | + 1 ml 5% Na-polyacrylat 20000/Fluka |
| PSS : | | + 2 ml 2% Na-styrensulfonat 70000/Aldrich |
| Sorbit : | | + 1 ml 10 % Sorbit |
| Ammonsulfat: | | + 1 ml 10 % Ammoniumsulfat |

**Beispiel 2.4:**

**Metalloxid-Überzüge mit inkorporierten Vitamin E (Tocopherol)**

Es wird eine Stammlösung aus 1 g des öligen Vitamin E und 10 ml Ethylglykol hergestellt ("Stammlösung"). 10 ml Metalloxid-Sol und 1 ml Stammlösung werden ggf. unter Zusatz eines Steuerstoffs gelöst und die klare Lösung auf Glanzkarton aufgetragen. Nach Trocknen an der Luft erhält man farblose, wischfeste Überzüge.

Die Freisetzung des öligen Vitamin E aus der Metalloxidmatrix wurde in folgender Weise bestimmt:
1x3 cm$^2$ des beschichteten Kartons wurden in einer Küvette mit 3 ml 30 % n-Propanol in Wasser behandelt und die Konzentrationen des freigesetzten Vitamin E in Abhängigkeit von der Zeit spektralphotometrisch (im Maximum bei 292 nm) bestimmt, vgl. Bild 13.

EP 0 680 753 A2

Tabelle 2.2

| Rezepturen | | |
|---|---|---|
| SiO$_2$: | 1 ml Stammlösung + 1 ml Sol 1A in 90 % Ethanol | |
| PDMDAAC:<br>Na-ascorbat:<br>DBPh<br>Paraffinoel: | wie oben, | + 1 ml 4 % Polydimethyldiallylammoniumchlorid in Wasser<br>+ 1 ml 10 % Natrium-ascorbat in Wasser<br>+ 0,1 g Dibutylphthalat<br>+ 0,1 g Paraffinöl AB |

Die Freisetzungskurven sind in Bild 13 zu sehen.

**Patentansprüche**

1. Komposit bestehend aus einem festen Matrixmaterial und mindestens einer in die Matrix inkorporierten Komponente, wobei das Matrixmaterial durch eine Metalloxidmatrix gebildet wird,
**dadurch gekennzeichnet, daß**
die Komponente homogen und molekular-dispers in der Matrix inkorporiert ist.

2. Komposit nach Anspruch 1, bei dem die inkorporierte Komponente aus einem freisetzbaren Wirkstoff und das Matrixmaterial aus SiO$_2$ besteht, welches anteilig durch Al$_2$O$_3$ oder MgO ersetzt sein kann, wobei das Komposit als volumenförmiges Material, insbesondere partikel-, granulat- oder tablettenförmig gebildet ist.

3. Komposit nach Anspruch 1 oder 2, **dadurch gekennzeichnet, daß** das Gewichtsverhältnis Wirkstoff : Matrix im Bereich 0.05:1 bis 1:1 liegt.

4. Komposit nach einem der Ansprüche 1 bis 3, **dadurch gekennzeichnet, daß** ferner mindestens ein Steuerstoff in die Metalloxidmatrix inkorporiert ist.

5. Komposit nach Anspruch 4, **dadurch gekennzeichnet, daß** das Gewichtsverhältnis Steuerstoff : Matrix im Bereich 0.05:1 bis 1:1 liegt.

6. Komposit nach Anspruch 4 oder 5, **dadurch gekennzeichnet, daß** als Steuerstoff:
   - niedermolekulare, wasserlösliche Stoffe wie Saccharid-Derivate, Salze oder Amide organischer Säuren oder Ammoniumsalze,
   - wasserlösliche polyanionische Verbindungen wie Na-Polystyrensulfonat, Salze der Polyacrylsäure, Carboxymethylcellulose, Dextransulfat oder polykationische Verbindungen wie Polydimethyldiallylammoniumchlorid,
   - mikroporöse Füllstoffe wie feste oder dispergierte Aerosile, oder
   - hochsiedende Flüssigkeiten wie Phthalsäureester, Glycerinether oder Paraffinöl enthalten sind.

7. Komposit nach einem der vorhergehenden Ansprüche, **dadurch gekennzeichnet, daß** der mindestens eine Wirkstoff durch flüssige oder feste Substanzen pflanzlichen oder organischen Ursprungs gebildet wird, welche in lebenden Organismen eine biologische Wirkung hervorrufen.

8. Komposit nach einem der vorhergehenden Ansprüche, **dadurch gekennzeichnet, daß** als Wirkstoff pharmazeutische Wirkstoffe, insbesondere einer der Wirkstoffe Alprazolam, Carbamazepin, Clomipramin, Clonidin, Detajmiumbitartrat, Diclofenac, Diazepam, Glibenclamid, Medazepam, Metoclopramid, Nifedipin, Pentoxifyllin, Prazosin, Talinolol, Trimipramin, Verapamil oder Vitamin K$_3$, enthalten sind.

9. Komposit nach Anspruch 1, durch das Metalloxidpulver oder -filme gebildet werden, die mindestens eine inkorporierte organische Flüssigkeit und/oder ein Öl enthalten.

10. Komposit nach Anspruch 9, das als Metalloxid SiO$_2$, Al$_2$O$_3$, TiO$_2$ oder deren Gemische enthält.

13

11. Komposit nach Anspruch 9 oder 10, bei dem die organischen Flüssigkeiten und/oder Öle bei Temperaturen höher als 100°C (bei Normaldruck von $10^6$ Pa) sieden.

12. Komposit nach einem der Ansprüche 9 bis 11, in dem das Gewichtsverhältnis Flüssigkeit: Metalloxid 0.01 bis 0.3:1 beträgt.

13. Komposit nach einem der Ansprüche 9 bis 11, in dem ein oder mehrere Penetrierungsmittel enthalten sind, wobei der Anteil der/des Penetrierungsmittel(s) bis zu 100 Gew.% bezogen auf Metalloxid beträgt.

14. Komposit nach Anspruch 13, in dem als Penetrierungsmittel:
    - niedermolekulare, wasserlösliche Stoffe wie Saccharid-Derivate, Salze oder Amide organischer Säuren oder Ammoniumsalze, oder
    - wasserlösliche polyanionische Verbindungen wie die Salze der Polystyrensulfosäure, Polyacrylsäure oder Carboxymethylcellulose oder polykationische Verbindungen wie Polydimethyldiallylammoniumchlorid enthalten sind.

15. Komposit nach einem der Ansprüche 9 bis 14, in dem die inkorporierte organische Flüssigkeit und/oder das Öl mit einer zweiten höhersiedenden Flüssigkeit gemischt ist.

16. Verfahren zur Herstellung von Kompositen bestehend aus einer Metalloxidmatrix und mindestens einem freisetzbaren Wirkstoff,
    **gekennzeichnet durch** die Schritte:
    (a) Herstellung eines in einem organischen, mit Wasser mischbaren Lösungsmittel gelösten Metalloxid-Sols, welches $SiO_2$, $SiO_2/Al_2O_3$ oder $SiO_2/MgO$ enthält,
    (b) Lösung des Wirkstoffes in dem Metalloxid-Sol, und
    (c) Gelieren durch Temperaturerhöhung oder pH-Änderung und/oder Entfernung des Lösungsmittels, wobei
    ein volumenförmiges Kompositmaterial gebildet wird.

17. Verfahren nach Anspruch 16, **dadurch gekennzeichnet, daß** die Herstellung des gelösten Metalloxid-Sols durch Hydrolyse eines Tetraalkoxysilans erfolgt, dem zur Modifizierung Anteile Metallalkoxyde zugesetzt werden können.

18. Verfahren nach Anspruch 16 oder 17, **dadurch gekennzeichnet, daß** der Lösung aus Wirkstoff und Metalloxid-Sol mindestens ein Steuerstoff zugesetzt wird, der die Wirkstoff-Freisetzung beeinflußt.

19. Verfahren zur Herstellung des Komposits nach einem oder mehreren der Ansprüche 9 bis 15, bei denen die organische Flüssigkeit oder ein Öl, ggf. in Gegenwart eines Penetrierungsmittels oder im Gemisch mit einer zweiten höhersiedenden Flüssigkeit, in einem wäßrig-organischen Metalloxid-Sol gelöst und anschließend das Lösungsmittel entweder unmittelbar aus dem Sol oder nach erfolgter Gelbildung aus dem Gel entfernt wird.

20. Verfahren zur Herstellung des Komposits nach einem oder mehreren der Ansprüche 9 bis 15, bei denen die organische Flüssigkeit oder ein Öl, ggf. in Gegenwart eines Penetrierungsmittels oder im Gemisch mit einer zweiten höhersiedenden Flüssigkeit, in einem wäßrig-organischen Metalloxid-Sol gelöst und anschließend mit der Lösung ein Schichtträger beschichtet wird.

21. Verwendung der Komposite nach einem der Ansprüche 1 bis 8 zur Herstellung von Arzneimittelzubereitungen, wie z. B. von Pulver, Dragees, Tabletten, Filmtabletten, Kapseln, Suspensionen, halbfesten Formen oder zur Herstellung von Arzneimitteln, die insbesondere Alprazolam, Carbamazepin, Clomipramin, Clonidin, Detajmiumbitartrat, Diclofenac, Diazepam, Glibenclamid, Medazepam, Metoclopramid, Nifedipin, Pentoxifyllin, Prazosin, Talinolol, Trimipramin, Verapamil oder Vitamin $K_3$ .enthalten.

22. Verwendung des Komposits nach einem der Ansprüche 9 bis 15 zur Herstellung von Arzneimittelzubereitungen, Kosmetika, Mitteln zur Körperpflege, bakteriostatischen und bakteriziden Mitten Insektiziden und Pestiziden und / oder zur Herstellung transdermaler Systeme..

# Carbamazepin-Freisetzung
## (aus Granulaten,bez. auf 200 mg Carbam.)

**Extinktion bei 285 nm**

Zeit/min

— Carbamazepin    —+— Carbamazepin ret    —*— C1 (1:1)
—□— C2 (1:2)    —△— C3 (1:4)

**Bild 1: Einfluß des Wirkstoff:SiO2-Verh.**

# Carbamazepin-Freisetzung
## (aus Granulaten,bez. auf 200 mg Carbam.)

**Extinktion bei 285 nm**

Zeit/min

— Carbamazepin    —+— C4 (Sorbit)    —*— C5 (PDMDAAC)
—□— C3 (ohne)    —◇— C6 (DBP)

**Bild 2: Einfluß von Zusätzen**

## Nifedipin-Freisetzung
### (Granulate,bez. auf 40 mg Nif.)

Bild 3: Unterschiede Sol-Gel-Varianten

## Nifedipin-Freisetzung
### (Granulate, bez. auf 40 mg Nif.)

Bild 4: Einfluß von Zusätzen

# Diclofenac-Freisetzung
## (Wirkstoffanteil 50 mg)

Bild 5: Einfluß der SIO2-Verkapselung

# Freisetzung von Wirkstoff-Basen
## (Wirkstoffanteil 30 mg)

Bild 6: Metalloxid-Verkapselungen
von Clomipramin (Cl) und Trimipramin (T)

# Vitamin K3-Freisetzung
## (Granulate, bez. auf 40 mg K3)

Bild 7: Freisetzung von Vitamin K3
in 50% n-Propanol

Bild 8: Limonen-Freisetzung (50° C)

Bild 9: alpha-Ionon-Freisetzung

**Bild 10:** Thermische Freisetzung aus SiO$_2$-Gelen (50° C)

**Bild 11:** Wirkstoff-Freisetzung aus Clomipramin-Filmen mit unterschiedlichen Metalloxiden

**Absorption bei 285 nm**

Legend:
— SiO₂        + +Harnstoff      * +PAA
□ +PSS        × +Sorbit        ◇ +Ammonsulfat

**Bild 12: Wirkstoff-Freisetzung aus Clomipramin-Filmen mit unterschiedlichen Penetrierungsmitteln**

**Absorption bei 292 nm**

Legend:
— SiO₂        * +PDMDAAC       □ +Na-ascorbat
× +DBPh       ◇ +Paraffinoel

**Bild 13: Wirkstoff-Freisetzung aus Vitamin E-Filmen mit unterschiedlichen Penetrierungsmitteln bzw. zweiter zugesetzter Flüssigkeit**